# EUROPEAN PATENT APPLICATION

(11) **EP 1 532 984 A1**
(43) Date of publication of application: **25.05.2005**
(21) Application number: 03292873.1
(22) Date of filing: 19.11.2003
(51) Int. Cl.: A61K 39/395, A61P 35/02, C07K 16/28

(54) **Use of anti CD44 antibodies for eradicating stem cells in acute myeloid leukemia**

(71) Applicant: INSTITUT NATIONAL DE LA SANTE ET DE LA RECHERCHE MEDICALE (INSERM), 75654 Paris Cédex 13 (FR); University Health Network, Toronto, Ontario M5G 2M9 (CA)
(72) Inventor: Smadja, Florence, 92260 Fontenay-aux-Roses (FR); Dick, John E., Toronto, Ontario M4E 2Z8 (CA)
(74) Representative: Peaucelle, Chantal

(57) **Abstract**

The present invention provides the use of an anti-CD44 antibody, a (Fab')2, Fab, Fab' fragment thereof, an IgG or IgM isotype thereof, in the preparation of a medicament for eradicating pathological stem cells in cancer therapy, and more specifically in acute myeloid leukaemia therapy.

## Description

The present invention relates to therapies against cancers, and more specifically against leukaemias.

Different types of leukaemia may be identified: lymphoblastic leukaemias, which particularly comprise acute lymphoblastic leukaemias (ALL) or lymphomas and myeloblastic leukaemias which particularly comprise acute myeloblastic leukaemias (AML). AML represents approximately half of the cases of leukaemia, i.e. approximately 1000 new cases a year in France and 6000 in the USA, with an incidence which increases exponentially over 40 years. AML corresponds to an inhibition of the differentiation of myeloid cells at an immature stage and is conveyed by invasion of the bone marrow and circulating blood by blastic cells, the cytological characteristics of which define the different AML sub-types classified M1 to M7 (French-American-British (FAB) classification), the most frequent being types M1 to M5.

In acute myeloid leukaemia (AML), the leukaemic clone is organized as a hierarchy originating from rare leukaemic stem cells (LSC) with extensive self renewal, which generate leukaemic blasts arrested at various stages of myeloid differentiation, defining the distinct AML subtypes.

In 1978, Leo Sachs published in *Nature* (1978, Aug 10; 274(5671):535-9) that mice leukaemic cells could be induced to differentiate in the presence of physiological growth and differentiation factors. This result was confirmed in human leukaemic cells and successfully transposed in vivo with two differentiation inducers of myelopoiesis, retinoic acid and G-CSF. Unfortunately, despite extensive research, complete remission is obtained in only two AML subtypes (AML3 and AML2 with t(8;21) translocation). Recently the inventors have shown (Nat Med. 1999 Jun; 5(6) :669-76) that ligation of CD44 reverses the different levels of myeloid differentiation blockage (AML1 to AML5). The differentiation of AML blasts was evidenced by:
- the ability to produce the oxydoreduction function such as oxidative burst,
- the increase expression of lineage antigens, and,
- cytological modifications, all specific of differentiated myeloid cells.
In addition, CD44 ligation with specific monoclonal antibodies (mAbs) can also induce terminal differentiation of THP-1, NB4 and HL60 cells lines, that are interesting models of AML5 (monoblastic subtype), AML3 (promyelocytic subtype)and AML2 (myeloblastic subtype) respectively. A massive apoptotic cell death could then be induced in NB4 cells but only a very moderate one in THP-1 and HL60 cells.

The leukaemic stem cells (LSC) are distinguished from all other AML cells by self renewal ability, i.e. the ability to generate daughter cells similar to the mother one. The extensive self-renewal ability is an intrinsic property of LSC, and has been shown essential for the development of leukaemia.

Experimentally, the human LSC are identified by transplantation in NOD/SCID immunodeficient mice in which they generate a disease faithfully recapitulating the AML type of the donor. Since they possess the ability to initiate the leukaemic clone on transplantation, they have been termed the SL-IC for SCID-Leukaemia Initiating Cells. These SLC are distinct from the other leukaemic cells because they are exclusively present within a CD34+cd38- cell fraction representing from 0.1% to 1% of the ALM cells population, and this is true in all AML subtypes.

In summary, in order for new therapies to cure AML, the LSC must be effectively targeted and eradicated.

The conventional treatment of AML is chemotherapy but, although it succeeds to induce an initial complete remission in 60-85% of patients, it is still unable to cure most AML patients (5-years survival rate: 37%) and only little progress has been made in the long-term survival of AML patients, especially in adults over 55-60 years (5-years survival rate: 15%). This situation has prompted efforts to develop new targeted therapeutic approaches, using anti-apoptotic agents (arsenic trioxide), anti-sense strategies (anti BCL2) and inducers of transcription (DNA methylases, histone acetylating agents). However, most therapeutic strategies currently employed, target cycling cells, and SL-IC are quiescent, indicating that new approaches must be found.

The inventors' work provides the first evidence that CD44 ligation by its own is sufficient to selectively eradicate pathological stem cells in vivo and is not toxic, establishing a new basis for developing CD44 targeted therapy in cancer.

Therefore, the present invention provides the use of an anti-CD44 antibody, a (Fab')2, Fab, Fab' fragment thereof, an IgG or IgM isotype thereof, in the preparation of a medicament for eradicating pathological stem cells in cancer therapy.

The invention further relates to the use of an anti-CD44 antibody, a (Fab')2, Fab, Fab' fragment thereof, an IgG or IgM isotype thereof, in the preparation of a medicament for purifying stem cells ex vivo in cancer therapy.
It may be noted that the term "antibody" covers, in the present application, the antibody but also any (Fab')2, Fab, Fab' fragment thereof, any IgG or IgM isotype thereof or any construction containing fragments thereof.

Another aspect of the invention concerns a method for eradicating pathological stem cells from a patient previously diagnosed as having pathological stem cells, comprising administering to said patient, an anti-CD44 antibody, a (Fab')2, Fab, Fab' fragment thereof, an IgG or IgM isotype thereof, under conditions allowing an antigen-antibody reaction, such that only pathological stem cells are eradicated.

Moreover the present invention further concerns a method for purifying stem cells ex vivo from a patient's tissue sample, said patient being previously diagnosed as having pathological stem cells, comprising contacting said tissue sample with an anti-CD44 antibody, a (Fab')2, Fab, Fab' fragment thereof, an IgG or IgM isotype thereof, under conditions allowing an antigen-antibody reaction. Such a method is adapted for purifying bone marrow cell populations.

Said medicament/method avoids the generation of pathological cells issued from pathological stem cells, in particular, leukaemic cells and cancer cells. Conventional therapies in AML are differentiation therapy. In these therapies, leukaemic cells whose differentiation is blocked, are stimulated to induce their differentiation. However, such a therapy need the use of a chemotherapy in order to eradicate leukaemic stem cells. The use of anti-CD44 antibody, according to the invention, induce the differentiation of leukaemic cells and the eradication of leukaemic stem cells without the requirement of any chemotherapy.

The invention is illustrated in the "examples" section below.

In the present invention, the anti-CD44 antibody is a polyclonal antibody, a monoclonal antibody or a synthetic peptide.

Advantageously, said anti-CD44 antibody is a human antibody, a mouse antibody or a rat antibody.

More specifically said antibody is a construction such as a chimerical antibody, preferably an humanized antibody, a ScFv construction, a CDR construction, a bispecific antibody, preferably produced by a quadrome.

The term "quadrome" refers to an hybrid-hybridome. (see article by Lebegue et al, "Production and characterization of hybrid monoclonal antibodies with IgG1/IgG3 double isotype", C R Acad Sci III. 1990;310(9):377-82) Preferably the invention comprises the use of P245 or A3D8 antibody. In particular, the quadrome may be realized from two anti CD44 antibodies, the first one being more specific for inducing the differentiation of leukaemic cells and the second one being more specific for the eradication of leukaemic stem cells.

In another preferred embodiment, said anti-CD44 antibody may be coupled with a toxin, a radioisotope, a cytotoxic molecule or with a galenic vector in order to improve the biodistribution, the half-life of the antibody or to help the transport of the antibody via formulation such as nanoparticles, nanocapsules, liposomes, preformed emulsions...

Examples of toxin, radioisotope, cytotoxic molecule are ricin, Yttrium 90, Iode 131, taxol, methotrexate adriamycine.

The medicament according to the invention may be administered at doses from approximately 10 mg to 1000 mg by cure, preferably in the order of 100 to 400 mg. The number of cures may be increased or reduced and/or repeated (over time) to optimise the efficacy of the medicament. Since the antibody used according to the invention is not generally toxic, its dosage may be adapted to the patient.

The production of the medicament may be in any suitable pharmaceutical formulation, and particularly in the form of tablets, granules, capsules, powder forms, suspension, oral solutions, solutions for injection. Administration may be preferably performed by slow infusion.

The medicament used according to the invention, may also comprise, in addition to the antibody coupled if necessary with one of the previously mentioned product, any suitable compound or excipient adapted to the desired formulation, particularly any pharmaceutically inert vehicle.

Advantageously, a suitable formulation is a saline solution for injection, preferably intravenous injection.

Pathological cells that can be treated by the medicament according to the invention are pathological stem cells, and more particularly leukaemic stem cells and breast cancer stem cells.

Indeed, there is increasing evidence that in other cancers, like in AML, the tumour clone is also maintained by the extensive proliferation and self-renewal of rare tumour stem cells. Since CD44 is also present in most cancer cells, CD44 ligation may be also efficient to eradicate such tumour stem cells, and thereby, it may have a therapeutic effect also in several cancers other than AML.

The present invention is illustrated by the following examples, given for purely illustrative purposes, which are in no way restrictive. The present invention also comprises any alternative embodiment that may be produced by those skilled in the art, without undue experimentation, from the disclosure given by the present application (including disclosure, examples and claims) and means according to the prior art.

### Example 1: Anti-CD44 monoclonal antibodies eradicate leukaemic (tumour) stem cells

NOD/SCID mice leukaemia model and transplanted PML-RAR mice are used. The NOD/SCID mice leukaemia model is a unique model, that faithfully recapitulates the pathology of *all subtypes* (except AML3) of human AML, and, most importantly, allows to monitor the fate of the very small subpopulation of human leukaemic stem cells, endowed with extensive proliferation and self-renewal capacity, and responsible for the maintainance of the leukaemic clone.

### Materials and Methods

**AML cells.** Fresh or frozen AML peripheral blood cells were enriched by Ficoll-density gradient centrifugation and washed in Iscove's Modified Dulbecco's medium (IMDM) containing 5% fetal calf serum.

**Transplantation of AML cells into NOD/SCID mice.** 8- to 12- week-old NOD/SCID mice are sub-lethally irradiated with 375 or 400 cGy from a ¹³⁷Cs source immediately before tail vein injection of AML cells. Mice receive human stem cell factor (SCF) and a fusion protein of huIL3/hu GM-CSF (PIXY321) every other day as intraperitoneal injections at a concentration of 10µg and 7µg per mouse, respectively.

**Assay for leukaemic stem cells (LSC).** It has been demonstrated (Bonnet and Dick, Nature Medicine 3:730-737, 1997) that the engraftment of AML into NOD/SCID mice results from proliferation and limited differentiation of a rare population of leukaemic stem cells (LSC), displaying a CD34++ CD38neg immunophenotype, that is present in the leukaemic clone and sustain it. Therefore, the success of the AML engraftment demonstrates the presence of LSC. At indicated time points (4-8 weeks), the percentage of leukaemic infiltration in bone marrow of transplanted NOD/SCID mice is evaluated by aspiration from the knee joint (average 10⁶ per aspirate) at different time points. The leukaemic population is labelled using a panel of mAbs to haematopoietic-specific antigens (CD45) and differentiation antigens (CD33, CD14, CD15, CD11b). The absence of CD19 is considered as indicator that the differentiated cells do not originate from *normal* haematopoietic stem cells comprised in the grafted AML sample.

### Results

**Table 1:**

| *P245 inhibits the development of AML stem cells in NOD*/*SCID mice. Mice were intravenously injected with 15.10*^{*6*} *human AML cells (day 0), and treated with P245 from day 20 to day 50* (*750µg*/*injection*, *3times per week). The % of human AML cells was measured in the bone marrow, on the basis of human pan-myeloid antigen huCD45 expression (aspiration from the knee joint, average 10*^{*6*} *cells per aspirate). Data are means +*/*-SD from 3 independent experiments, 5 mice* /*group. This table shows that P245 inhibits the development of AML.* * *secondary recipients did not receive P245 injection* | | | | | |
|---|---|---|---|---|---|
| | | % huCD45+ cells | | | |
| | | in primary recipients | | in secondary recipients* | |
| Patient | AML subtype | untreated | P245-treated | From untreated primary recipient | from P245-treated primary recipient |
| 4971 | M5 | 23+/-19 | 0 | 23.4+/-16 | 0.0+/-0.B0 |
| 5131 | M5 | 67+/-20 | 7+/-10 | 1.7+/-1.5 | 0.0+/-0.1 |
| 5173 | M4 | 14+/-12 | 2+/-2 | 7.3+/-3 | 0+/-0 |

The four independent experiments performed so far clearly show, in a very reproducible manner, that P245 is highly efficient to eradicate most AML cells in the primary recipients (table 1). This may be partly due to the induction of terminal differentiation, as shown in table 2. However, it is also, and probably mainly, due to the eradication of most leukaemic stem cells, as shown by secondary transplantation assays (table 1). These results show that it is possible to eradicate AML stem cells *in vivo,* and it should be pointed out that no toxicity nor other undesirable side-effect was observed. The effect of P245 on long-term survival was further investigated. In addition, the effect of P245 on normal stem cells was also studied. Most interestingly, in a preliminary experiment, no inhibitory effect of P245 on the engraftment of normal CD34+ cord blood cells was observed, showing that P245 selectively eradicate AML stem cells in vivo.

**Table 2.**

| *In vivo differentiation of AML blasts in P245-treated primary recipients: Differentiation* is *evidenced by increased expression of the granulocytic-specific differentiation antigen CD15*, *on the AML cells (CD45+) . This experiment is one representative of 4 independent experiments.* | | | |
|---|---|---|---|
| Patient | Treatment | % huCD45+ cells | %CD15+in the CD45+ population |
| 4971 | No | 43 | 22 |
| | P245 | 6.2 | 56 |

The transplanted PML-RAR mice, has allowed the inventors to investigate the in vivo effect of CD44-targeted molecules on a model of AML3 subtype, the only one which can not be engrafted into NOD/SCID mice. Since mAbs to murine CD44 was not at disposal, the therapeutic efficacy of HA was investigated, and compared to the one of retinoic acid, which induces full terminal differentiation of AML blasts and full remission of the transplanted PML-RAR mice. The results obtained (summarized in table 3), clearly show that, after 4 days of administration, HA is as efficient as retinoic acid to abrogate the splenomegaly characteristic of the disease, and it also succeeds to decrease leukaemic blast infiltration in the bone marrow. This effect is HA-dose dependent. The apparition of differentiated granulocytic precursors strongly suggest that HA induces terminal differentiation of AML blasts, as it does in vitro, and similarly to retinoic acid. Collectively these results show that for the first time CD44 ligation is an efficient means to eradicate AML cells in vivo and provide a new basis for developing CD44 targeted therapy in AML.

**Table 3:**

| *HA inhibits growth and induces terminal differentiation of PML-RAR cells (AML3) in vivo HA (6.105kAa) was administered through an osmotic pump, at a rate of 1µl*/*hour for 4 days, into leukaemic mice, engrafted 12 days before with 10*^{*5*} *leukaemic PML-RAR blasts. A strong inhibition of splenomegaly is observed, associated with a decrease of blastic infiltration (enumerated by microscopic observation) in the bone marrow and an increase of differentiating granulocytic precursor cells. Data are means +*/*-SD from 3 independent experiments, 5 mice* /*group*. *RA: retinoic acid* | | | |
|---|---|---|---|
| Treatment | mean spleen weight (mg) | % blasts in bone marrow | % myelocytes plus metamyelocytes in bone marrow |
| No | 480+/-45 | 85+/-7 | 12+/-3 |
| HA | 120+/-57 | 28+/15 | 48+/-23 |
| RA | 135+/-68 | 18+/-7 | 67+/-17 |

### Example 3: Formulation of a galenic vector - emulsion

The LIPOID E-80, Vit E and stearylamine are dissolved directly in the oil phase. Whereas the poloxamer and glycerol are directly dissolved in the aqueous phase. The oil and aqueous phases are prepared separately using a magnetic stirrer, filtered and heated to a temperature of 70°C. The two phases are mixed using a magnetic stirrer. The temperature is brought up to 85°C. The mixture is homogenized with Polytron or Ultraturrax for 3 to 5 min. The temperature is decreased rapidly to 20°C. The emulsion is passed trough a high-pressure homogenizer (microfluidizer) for 5 min. The temperature is brought rapidly to 20°C. The pH is adjusted to the desired value with 0.1M hydrochloric acid. The emulsion is filtered through a 0.45µm filter, stored under nitrogen atmosphere in siliconized glass bottles and sterilized in an autoclave.

It was shown that up to 40 molecules of IgG could be conjugated to one single oil cationic droplet.

In conclusion, this galenic vector was shown to increase the number of antibody sites on AML cells and to improve the half-life of anti CD44 antibodies.

### Example 4: Treatment according to the invention

Formulation of the medicament (flask of 20mL, lyophilised):
- active principle : 100 mg of lyophilised P245
- excipients : saccharose, polysorbate, monosodic phosphate, disodic phosphate.

This formulation may be kept between +2 and +8°C, in its packaging for 18 months. Do not freeze.

Once prepared, the medicament may be preserved only 3 hours.

Treatment: 5mg/Kg are injected by slow infusion (for example, during two hours).

## Claims

1. Use of an anti-CD44 antibody, a (Fab')2, Fab, Fab' fragment thereof, an IgG or IgM isotype thereof, in the preparation of a medicament for eradicating pathological stem cells in cancer therapy.

2. Use of an anti-CD44 antibody, a (Fab')2, Fab, Fab' fragment thereof, an IgG or IgM isotype thereof in the preparation of a medicament for purifying stem cells ex vivo in cancer therapy.

3. The use according to claim 1 or 2, wherein said anti-CD44 antibody is a polyclonal antibody, a monoclonal antibody or a synthetic peptide.

4. The use according to any of the previous claims, wherein said anti-CD44 antibody is a human antibody, a mouse antibody or a rat antibody.

5. The use according to any of the previous claims, wherein said anti-CD44 antibody is a construction.

6. The use according to claim 5, wherein said anti-CD44 antibody is a chimerical antibody.

7. The use according to claim 6, wherein said chimerical anti-CD44 antibody is a humanized antibody.

8. The use according to claim 5, wherein said anti-CD44 antibody is used in a ScFv or a CDR construction.

9. The use according to claim 5, wherein said anti-CD44 antibody is bispecific.

10. The use according to claim 9, wherein said bispecific anti-CD44 antibody is produced by a quadrome.

11. The use according to claim 3, wherein said anti-CD44 antibody is P245 or A3D8.

12. The use according to any of the previous claims, wherein said anti-CD44 antibody is coupled with a toxin, a radioisotope, a cytotoxic molecule or with a galenic vector.

13. The use according to any of the previous claims, wherein said anti-CD44 antibody is administered by slow infusion at doses from 10mg to 1000mg by cure.

14. The use according to any of the previous claims, wherein said stem cells are leukaemic stem cells.

15. The use according to any of the previous claims, wherein said stem cells are breast cancer stem cells.
